# EUROPEAN PATENT APPLICATION

(11) **EP 2 060 987 A2**
(43) Date of publication of application: **20.05.2009**
(21) Application number: 09002118.9
(22) Date of filing: 21.12.2005
(51) Int. Cl.: G06F 19/00

(54) **System and method for managing restorative care**

(30) Priority: 21.12.2004 US 638007 P
(62) Divisional of application: 05854986.6
(71) Applicant: Q-Trac, LLC, Franklin, IN 46131 (US)
(72) Inventor: Summers, Jennifer R., Franklin, Indiana 46131 (US); Kirby, Lana B., Franklin, Indiana 46131 (US); Kirby, Timothy V., Franklin, Indiana 46131 (US)
(74) Representative: Findlay, Alice Rosemary

(57) **Abstract**

An automated system and method for managing restorative care programs in a long-term care environment (10, 10'. 10", 10"') is provided. In one embodiment, the system (10, 10'. 10", 10"') includes a device or process for generating an up-to-date assignment sheet (300, 310, 320) upon detecting a change in a resident's condition. In one embodiment, the device or method includes a feature of sending a communication to a caregiver when an assignment sheet changes (280). One embodiment provides for the monitoring of restorative care administered to residents and/or infection reporting (400, 500, 600) to facilitate payment or reimbursement for the provision of restorative care.

## Description

### Cross Reference

This application claims the benefit of U.S. Provisional Patent Application Serial No. 60/638,007, entitled "System and Method for Managing Restorative Care," filed on December 21, 2004, and the same is expressly incorporated herein by reference.

### Background

The present invention relates to the field of computer software for management of resident care in long-term care facilities, skilled nursing facilities and the like (referred to herein as "long-term care facilities" or "long-term care environment" for ease of discussion), and for managing restorative care therein.

In the United States and other countries, the way that nursing and medical care is provided to residents of long-term care facilities may be subject to government regulations. Restorative treatment of residents of long-term care facilities may be mandated by these regulations. In the United States, these regulations are often enforced routinely, and failure of a facility to comply with regulations may result in assessment of fines and/or loss of the facility's eligibility for reimbursement or payment for the provision of restorative care.

In general, a restorative program is a treatment program or nursing program that is directed to restoring or maintaining an individual's physical, mental, and/or psychosocial functioning at the highest practicable level. Examples of restorative programs include treatments and/or therapies relating to maintaining or improving an individual's hygiene, ambulation, eating, swallowing, communication, toileting, mobility, range of motion, care of prosthesis or other medical devices, or other abilities or functions.

Restorative programs may be provided by a variety of different care providers or caregivers, including registered nurses or RNs, licensed practical nurses or LPNs, social workers, certified nursing assistants or CNAs, social services workers, licensed physical therapists, hospital or nursing home activity staff, and others under the supervision of RNs and LPNs. These care providers are in need of a way to efficiently manage their restorative care programs.

### Summary

According to one embodiment of the present invention, there are provided methods and systems for long-term care facilities and skilled nursing facilities to create daily assignment sheets for multiple rooms and patients for use by caregivers in a clear, concise, readable, and real time summary format containing information relative to residents' care needs.

According to another embodiment of the present invention, there are provided methods and systems for facilitating reimbursement or payment for restorative care provided by caregivers including providing supporting chart documentation for claiming reimbursement or payment.

According to another embodiment of the present invention, there are provided methods and systems for monitoring resident progress, maintenance, or decline in a restorative care program, and methods and systems to prompt caregivers through alerts to periodically review results of restorative care programs and allow easy retrieval and access for modification as a resident's needs and condition changes.

According to another embodiment of the present invention, there are provided methods and systems for automated, real-time planning and management of restorative programs for long-term care facilities. Caregiver assignment sheets are updated or modified continuously and immediately in real-time as a resident's condition changes.

According to another embodiment of the present invention, there are provided systems and methods which track and monitor infections, immunizations, tests, and/or changes in treatment or therapy for residents and automatically alerts a caregiver when a resident requires an immunization test, treatment for an infection, therapy or other restorative program.

According to a further embodiment of the present invention there are provided methods and systems for managing restorative care management by level, such as CNA, Restorative Aide (a CNA that has been specifically trained for restorative care). Care level is a factor in determining resident progress and staffing needs. For example, Level I is formalized physical therapy performed by a licensed physical therapist, Level II is care by a specially-trained Restorative Aide, and Level III is restorative care performed by a CNA.

In addition, further embodiments of the present invention audit the use and effectiveness of assistive devices and equipment such as siderails, restraints, and alarms.

One aspect of the present invention includes creating, generating, and providing daily assignment sheets for caregivers in long-term care facilities or skilled nursing facilities. Another aspect of the present invention includes providing systems and methods for implementation, documentation, and periodic review of restorative care programs for individuals in such facilities. A further aspect of the present invention includes providing methods and systems of infection reporting and control for residents of such facilities. Still another aspect of the present invention includes providing reports used by decision makers in long-term care facilities for monitoring the daily care and treatment of residents in such facilities.

In one embodiment of the present invention, a method for managing restorative care in a long-term care facility is provided. The method includes the steps of receiving a resident identifier identifying a resident of a long-term care facility, creating a restorative program including a description of a resident's condition, a goal for maintaining or improving the residents' condition, and a plan for achieving the goal, assigning one of a plurality of levels to the restorative program, linking the restorative program to the resident identifier, storing the resident identifier, level, and restorative program in a computer-readable medium, repeating the above receiving, creating, assigning, linking, and storing operations for a plurality of residents of a long-term care facility, receiving data relating to at least one of a work shift, caregiver, section of a facility, and grouping of residents, generating an assignment sheet for one of a work shift, caregiver, section of a facility, and grouping of residents, the assignment sheet including resident care data for a plurality of residents, the assignment sheet including at least a portion of the restorative programs for the residents, automatically updating the assignment sheet in real time as a resident's condition changes; and alerting a caregiver to the updated assignment sheet.

The method may further include the steps of receiving infection information, linking the infection information and the resident identifier, and sending a caregiver alert when the infection information linked to the resident identifier exceeds a threshold.

The method may include the steps of receiving input indicating changes to the restorative program, storing the changes in a computer readable medium, and sending a communication to a caregiver based upon the changes to the restorative program.

The method may include the step of automatically generating a periodic report including the resident identifier, the goal and the accomplishment data. The periodic report may be generated at least monthly. The method may include the step of sending at least a portion of the data in the report to a remote computer.

The method may include the steps of receiving a plurality of infection information inputs, storing the plurality of infection information inputs, and sending a caregiver alert when the number of stored infection information inputs exceeds a limit.

The assignment sheet may be automatically updated if the work shift, caregiver, section of the facility, or grouping of residents changes.

Also in accordance with the present invention, a method for facilitating reimbursement or payment for restorative care programs in long-term care facilities is provided. The method includes the steps of receiving predetermined minimum restorative care data from a remote computer; storing the minimum restorative care data in a memory, creating a restorative care program including the minimum restorative care data for a resident of a long-term care facility; receiving resident care data for the resident via an input device; storing the resident care data in a memory, comparing the resident care data to a restorative care program for the resident updating the restorative care program in real time based on one or more of the resident care data; receiving an update to the minimum restorative care data from a remote computer; updating the restorative care program based on the update; repeating one or more of the above steps for other residents of the long-term care facility; and generating a report indicating at least one of restorative care provided by the facility and residents' progress against their respective restorative care programs.

The method may include the step of automatically detecting a change in the resident's status based on a change in the resident care data. The method may include the step of notifying a caregiver of a change in a resident's restorative care program in real time. The method may include the step of periodically prompting a caregiver to review a restorative care program for a resident, and discontinuing the prompting once a response is received.

Further in accordance with the present invention, a method is provided, including the steps of maintaining a database including an resident identifier, a restorative care program record associated with the identifier and an infection record associated with the identifier; receiving input indicating a change in one of the restorative care program record and the infection record; modifying the database effective to store the change in one of the restorative care program record and the infection record; providing a first notice to a caregiver based upon the change to the restorative program; and providing a second notice to a caregiver based upon the infection record exceeding an infection frequency.

The method may include the step of providing a third notice based upon a plurality of infection records associated with a plurality of resident identifiers. The step of providing a first notice may include displaying a message on a display of a computer workstation. The step of providing a second notice may include sending an electronic mail message.

The method may include the steps of receiving input designating a goal associated with a restorative care program; and storing data indicating progress toward the goal. The method may include the step of generating a report including the data indicating progress toward the goal. One or more of the steps of maintaining, receiving and modifying may include processing information on a networked computer. The method may include the steps of discontinuing a restorative care program; and storing a record of a reason for discontinuing the restorative care program.

Additional aspects and features of the present invention will become apparent to those skilled in the art upon consideration of the following detailed description, accompanying drawings, and appended claims.

### Brief Description of the Drawings

Figs. 1A-1D are simplified schematic diagrams of exemplary configurations of restorative care management systems in accordance with the present invention.
Fig. 2A is a flow diagram illustrating operation of a restorative care program module according to one embodiment of the present invention.
Fig. 2B is a flow diagram illustrating operation of an assignment sheet program module according to one embodiment of the present invention.
Figs. 3A and 3B are exemplary assignment sheets.
Fig. 4 is a flow diagram illustrating operation of a restorative care monitoring and update program module according to one embodiment of the present invention.
Fig. 5 is a flow diagram illustrating operation of an infection monitoring program module according to one embodiment of the present invention.
Fig. 6 is a flow diagram further illustrating operation of an infection monitoring program module according to one embodiment of the present invention.
Fig. 7 is a simplified schematic diagram of an embodiment of the present invention including an interface to an external system.
Fig. 8 is an exemplary user interface for inputting resident information.
Fig. 9 is an exemplary user interface for creating a restorative care program.
Fig. 10 is an exemplary user interface for generating an assignment sheet.
Fig. 11 is an exemplary user interface for reporting infection information.

### Detailed Description

For the purposes of promoting an understanding of the principles of the present invention, reference will now be made to the embodiments illustrated in the drawings and specific language will be used to describe the same. It will nevertheless be understood that no limitation of the scope of the present invention is thereby intended, such alterations and further modifications in the illustrated embodiments, and such further applications of the principles of the present invention as illustrated therein being contemplated as would normally occur to one skilled in the art to which the present invention relates.

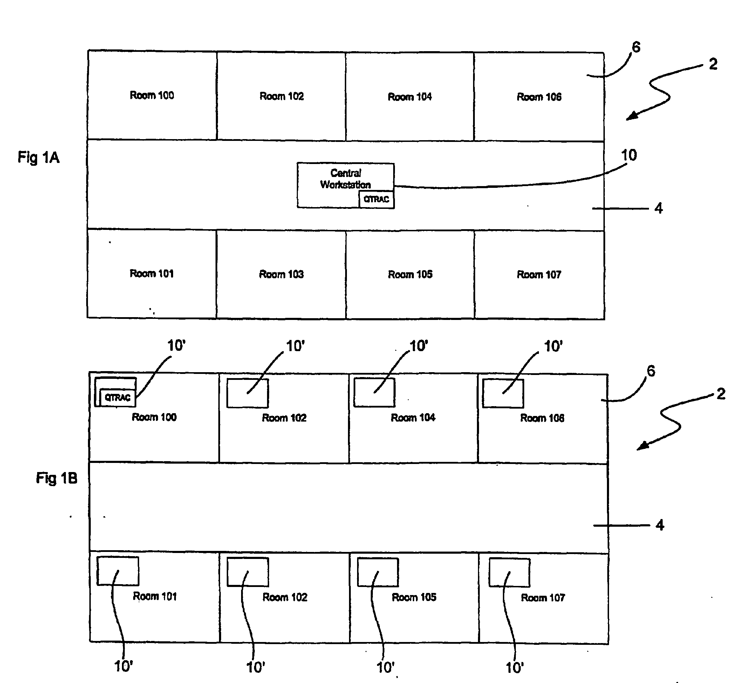

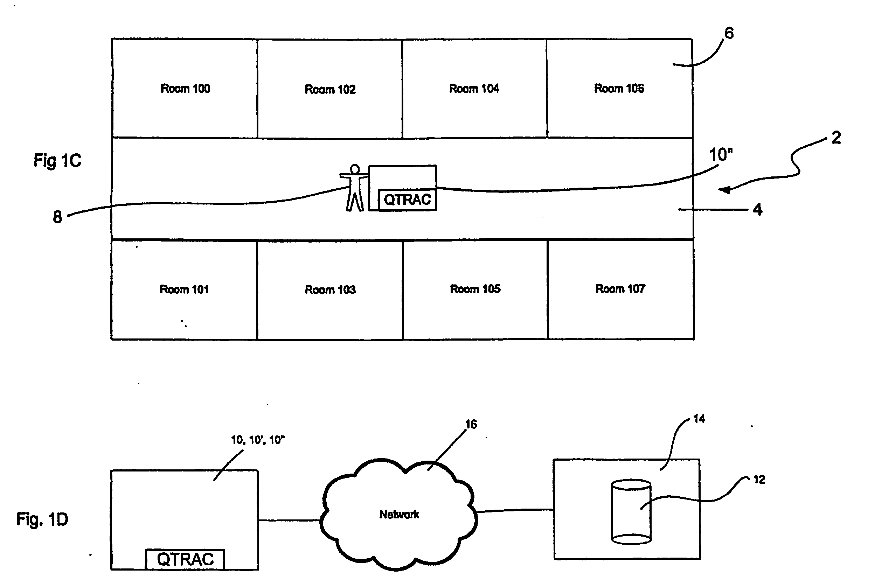

With reference to Figs. 1A-1D there are shown various configurations of a restorative care management system in accordance with the present invention. Fig. 1A shows a configuration of a restorative care management system in which a single central computer workstation 10 is located in a long-term care facility 2 or a wing, ward, section, or floor 4 of a long-term care facility 2. The central workstation 10 manages restorative care for residents of the one or more rooms 6 of the facility 2 or portion thereof. The central workstation 10 may be a standalone computer system or may be connected to one or more other computer systems, servers, or processing devices via one or more internal and/or external computer, cable, telephone, or wireless networks, for example, as shown in Fig. 1D.

With reference to Fig. 1B, another configuration of a restorative care management system in accordance with the present invention is shown. In the configuration of Fig. 1B, each room 6 includes a computer workstation 10' for operating the restorative care program management system of the present invention. Each of the workstations 10' may be operably linked to one another and/or to one or more servers by a suitable network and may be connected to one or more other computer systems, servers, or processing devices via one or more internal and/or external computer, cable, telephone, or wireless networks, for example, as shown in Fig. 1D.

With reference to Fig. 1C there is shown another configuration of a restorative care management system in accordance with the present invention. In the configuration of Fig 1C the restorative care management system includes one or more portable and/or handheld computer or processing devices 10" which are used in operating the restorative care management program of the present invention. Device 10" may be a personal data assistant, laptop or notebook computer, cell phone, badge, tag or pendant, or other similar suitable processing device configured to travel with or be carried by a caregiver or to travel with or be carried by a resident 8. Device 10" can be operatively coupled to a wireless network, or may be coupled to a network at one or more docking stations, for example.

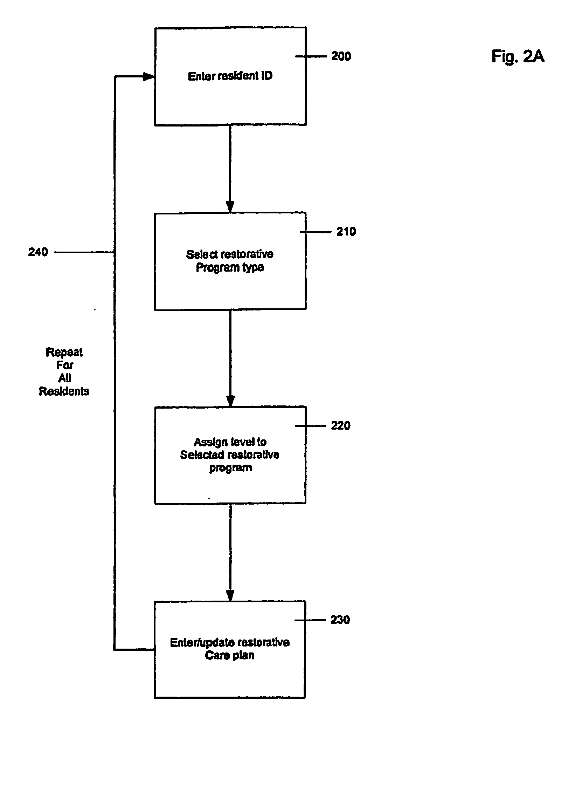

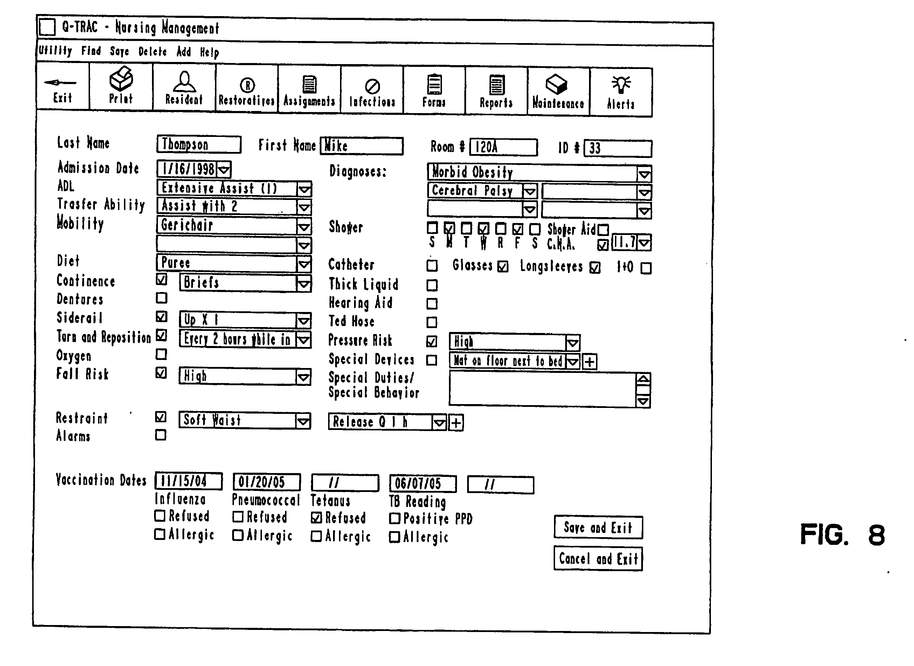

Each of the systems or devices 10, 10', 10" may be operably coupled to a network 16. As shown in Fig. 1D, all or a portion of the restorative care management system in accordance with the present invention may be stored on or operated from a remote device 14. The remote device 14 may be another computer or processing device located within the facility 2 or a computer, system or device located outside of or offsite from the facility 2. With reference to Fig. 2A there is shown a flow diagram illustrating operation of a restorative care program module according to one embodiment of the present invention. The flow diagram of Fig. 2A begins at operation 200. At operation 200, a caregiver or other person authorized to input data inputs a unique resident identifier (such as an identification number) using a computer mouse, stylus, keypad, keyboard, microphone, or other suitable input device. An exemplary user interface for entering resident information is shown in Fig. 8. The unique resident identifier may be automatically generated. A variety of additional information about the resident, the resident's condition, and/or the resident's care protocol, may also be input at operation 200. For example, a resident's last name, first name room number, admission date (which may be set to default to the current date or altered), and diagnoses may be entered. Additionally all or some of the following information may be input. Further, if the user needs to enter information which is not contained within the designated menu, the user has the option to manually enter the information in any of these areas.

Information about the amount of assistance required for activities of daily living may be entered. A menu may be provided for entry of the following categories with respect to a variety of activities: assist, extensive assist, independent, minimal assist, setup help, supervision, total, or total dependent.

Information about a resident's transfer ability may be entered to record how the resident moves between surfaces, to or from a bed, chair, wheel chair, standing position, or other locations. For various transfer situations a menu may be provided to designate a residents transfer ability as requiring a three person lift, assistance of one, assistance of two, a hoyer lift, a mechanical lift where the resident is transferred via mechanical assistance, self lifting, or setup help.

Information about a resident's mobility may be entered. A menu may be provided to designate that the resident uses a cane, gerichair, merry walker, scooter, electric wheelchair, self ambulates, requires standby assist, a walker, or a wheelchair.

Information about a resident's diet may be entered. A menu may be provided for entry of an indication a user is diabetic, requires mechanical soft food, can have no added salt, can have no concentrated sweets, requires puree, or tolerates a regular diet.

Information about a resident's continence may be entered. A menu may be provided for entry of an indication that a resident is continent, incontinent, requires briefs, pads, or pull-ups.

Information about a resident's denture needs may be entered. A menu may be provided for entry of an indication the resident has upper dentures, lower dentures, or both, or that the dentures are partial.

Information about a resident's requirements for a siderails may be entered. A menu may be provided for entry of an indication the resident requires full siderails, half siderails, or padded siderails and how and when the siderails must be used.

Information about a resident's requirements for turning and repositioning may be entered. A menu may be provided for entry of an indication that the resident requires turning and repositioning every hour while in bed or every two hours while in bed.

Information about a resident's requirements for restraints may be entered. A menu may be provided for entry of an indication that the resident requires a lap buddy, lap tray, quick release, soft waist restraint, side rails, a vest or shoulder harness. For required restraints a menu may be provided to indicate how long the restraint is to occur.

Information about a resident's requirements for alarms may be entered. A menu may be provided for entry of an indication that the resident requires a bed alarm, bed pad alarm, or personal safety alarm. For a required alarm, a menu may be required that indicates the alarm is to be used at all times, in bed, in bed and in a wheelchair, in a recliner or in a wheelchair.

Information about a resident's requirement for thick liquids may be entered. A menu may be provided for entry of an indication that the resident is restricted from or requires honey, nectar, pudding, or has a fluid restriction.

Information about a resident's requirements for special devices may be entered. A menu may be provided for entry of an indication that the resident requires hand rails in the bathroom, a low bed, a mat on the floor next to the bed a non-skid surface wheelchair cushion, a noodle in bed, a pressure relief cushion, splints, wheelchair anti-tippers, or a wedge pommel cushion.

Information about a resident's vaccination requirements and due dates may be entered. A menu may be provided for entry of the date a TB site was read, the date the next TB test is due, whether the resident has a positive PPD reading, whether the resident is allergic to the TB test, the date and year the influenza vaccine was given, the date and year the pneumococcal vaccine was given, the date and year the tetanus vaccine was given.

Indications that the resident: requires TED hose and when they are to be worn, hearing aids and for which ear, requires oxygen, is a fall risk, has a pressure ulcer risk and the level of the risk, has a catheter, requires glasses, requires long sleeves, requires monitoring of their intake or output, or the frequency with which or days of the week on which the resident requires showering also be input through one or more menus. A menu for entry of special notes or notation of special behavior can also be provided.

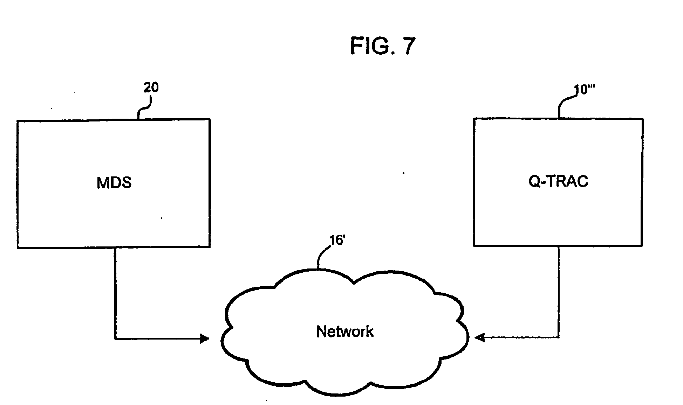

As described above, some or all of the foregoing and other information may be entered manually through a menu driven interface including one or more menus, such as shown in Fig. 8. The menu or menus may also allow a user to enter information not listed on the menu or menus. Some or all of the foregoing and other information may also be imported from another computer system, such as a minimum data set or MDS system, via a software interface as shown in Fig. 7, described below. The information input at operation 200 is received by the system and is stored and maintained in a database or other suitable storage medium. The same or similar process can be repeated for additional or all residents of a particular long term care facility.

At operation 210, data relating to and/or describing the type of restorative program or programs to be created for the resident identified in operation 200 is selected. Each resident may have assigned to them one or more restorative care programs, or restoratives, relating to one or more predefined restorative care areas. Examples of predefined restoratives include ambulation, amputation and prosthesis care, active range of motion, bed mobility, communication, eating, hygiene, passive range of motion, splint or brace requirement, swallowing, toileting, communication/sensory stimulation, exercise, grooming, and others. Custom restorative care programs may also be assigned to a user. One or more predefined or custom restorative care programs can be linked to a resident identifier.

A level is assigned to each restorative care program at operation 220. A menu is provided in which Level I, Level II or Level III can be input for a particular restorative care program. Level III indicates that the restorative is implemented by a certified nurse assistant or CNA. Level II indicates that the restorative is implemented by a restorative aide. Level I indicates formalized therapy performed by a licenses therapist. Since Level I presents unique staffing needs it may be omitted from operation 220 and included in a separate therapy program module.

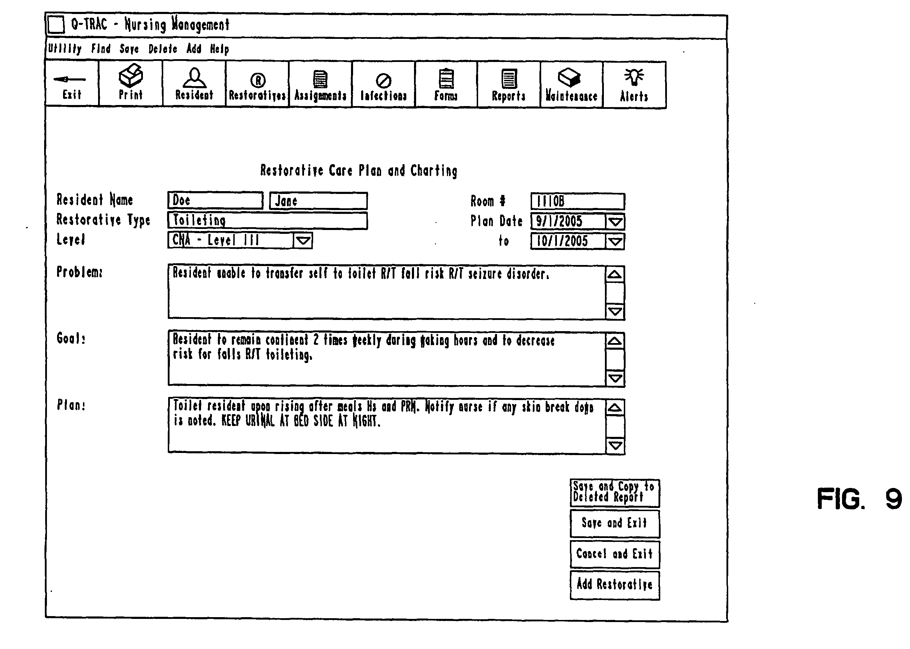

After the level is assigned, the restorative care program is created for the resident, or updated, at operation 230. An exemplary user interface for creating a restorative care program is shown in Fig. 9. At operation 230 fields are provided for entry or modification of a problem, a goal and a plan. The problem field denotes a resident condition that is a subject of a resident's restorative care program, for example, resident noted with decreased joint mobility upper extremities. The goal field denotes one or more measurable objectives of the restorative care program, for example, resident to maintain current joint mobility status until next review. The plan field denotes specific interventions that will be taken to meet the objectives identified in the goal field, for example, provide passive range of motion to bilateral upper extremities ten repetitions two times daily; discontinue when point of resistance is met; discontinue if resident complains of pain and notify therapy if decrease in range of motion is noted.

The system also allows for receiving information relating to how long, for example, how many minutes per day, certain restorative care is given to a resident. Such information is stored in memory and may be monitored, tracked, and reported on in order to facilitate a long term care facility's ability to receive reimbursement or payment for the restorative care provided.

Additionally, the system allows an indicator of a resident's progress toward a restorative program goal to be input, stored monitored and reported on. In the illustrated embodiment, a plurality of codes are used to indicate progress, for example: M = Met goal fully (100%); G = Met 60% of goal; P = Met less than 50% of goal; R = Refusal to meet goal. Such information may also be monitored and reported in order to facilitate payment or reimbursement for restorative care provided by the facility.

Once input is provided for the problem, goal and plan fields, operation 230 allows the user to save the restorative care program and exit the module, to cancel the restorative care program and exit the module, or to add an additional restorative program. The restorative care program can also be printed or electronically transmitted for inclusion in a resident's chart.

Based upon the requirements of a restorative care program, automated cues, notices, or alerts, such as electronic mail messages, pager messages, text, audio or visual signals, may be generated and transmitted to specified caregivers of to specific devices in a restorative management care system on a periodic basis, for example, daily, weekly, or monthly, or upon occurrence of an event such as a change in resident's status to trigger the caregiver to review and update the resident, restorative or plan information.

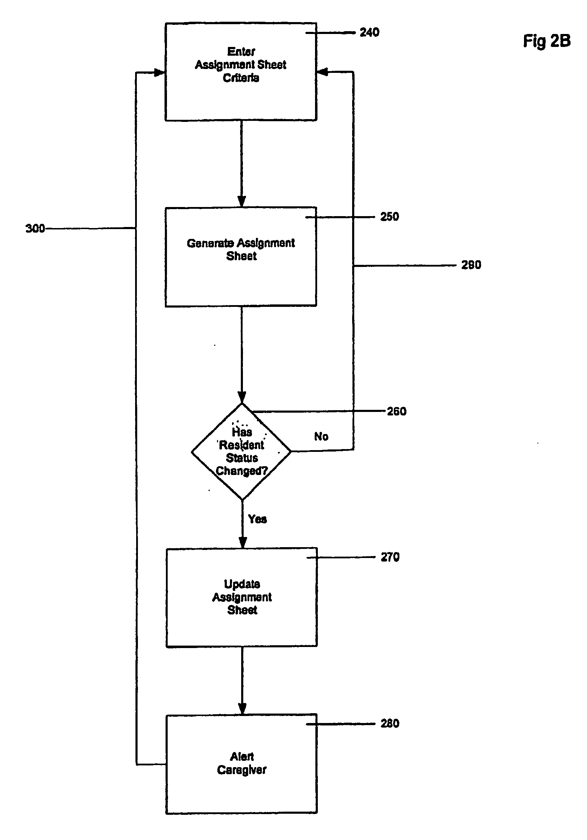

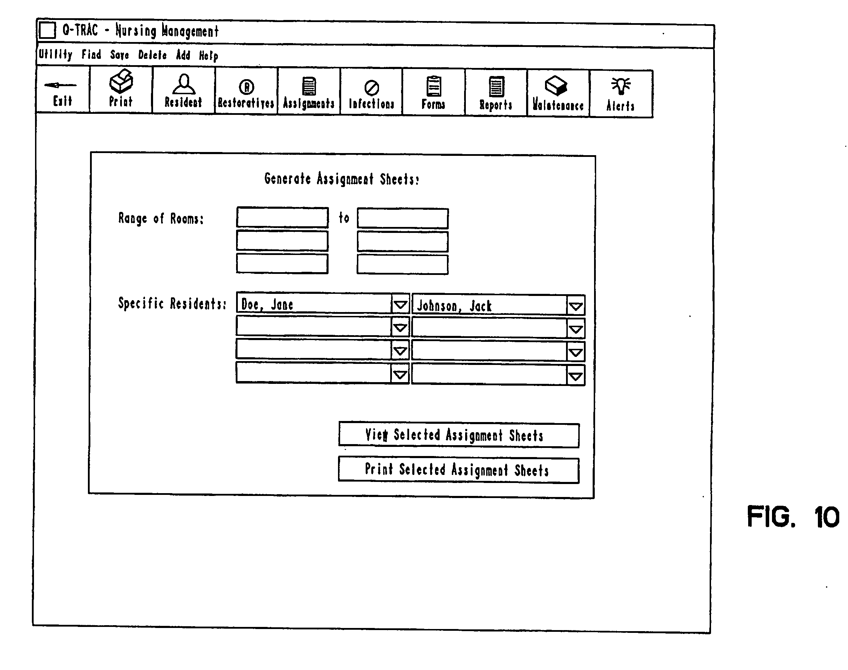

With reference to Fig. 2B there is shown is a flow diagram of an assignment sheet program module according to one embodiment of the present invention. Assignment sheets can present a clear, concise, readable, user friendly summary of essential information pertaining to residents' care in a variety of formats. An assignment sheet may be generated for one or more portions or segments of a long-term care facility, or for the entire facility. For example, an assignment sheet may be generated for each work shift as new caregivers arrive. As another example, an assignment sheet may be generated for a specific caregiver, or for a grouping of residents in a certain wing, floor, or section of the facility. Assignment sheets may also be generated for a specific set or range or rooms for a shift, day or week, or for a set of rooms plus a set of one or more residents. Furthermore, assignment sheets can be generated for caregivers who are working on a substitute or temporary basis due to a shortage of scheduled staff. Fig. 10 shows an exemplary user interface for generating assignment sheets.

At operation 240 of Fig. 2B, the criteria to be used in generating one or more assignment sheets, such as the foregoing or other criteria, is input. The module then proceeds to operation 250. At operation 250, one or more assignment sheets are generated automatically based on the criteria entered in operation 240. Computer programming logic is used to determine the data to be used from the database and inserted into the assignment sheet. All or portions of the assignment sheet or sheets may be communicated to one or more caregivers in a variety of ways including paper printouts, electronic mail, transmission to a handheld or portable computing device, or annunciated via a speaker/intercom system or other voice transmission system. The module then proceeds to conditional 260.

Conditional 260 tests whether a resident's status has changed. Programming logic monitors to detect when resident information is changed, added or deleted, for example, at operation 200 of Fig. 2A or from other input to the database records associated with a resident. If conditional 260 determines that a resident's status has not changed, the process then returns to operation 240 via line 290 and repeats operations 240 and 250 or may wait until a status change is detected. If conditional 260 determines that a resident's mental, physical, and/or psychosocial condition has changed, for example based upon information input into a database, the module proceeds to operation 270.

At operation 270 updating of an assignment sheet is performed. The data included in the assignment sheets is updated automatically, using computer programming logic, immediately when a resident's condition changes or is added or deleted. From operation 270 the module proceeds to operation 280.

Operation 280 automatically generates a notice, or alert for one or more caregivers when an assignment sheet is updated. Computer programming logic generates and transmits an alert to one or more designated caregivers by email, voicemail, pager, audio or visual signal, or other suitable alerting means. Providing an automatic update allows a caregiver to receive a variety of information immediately in real time.

Examples of the information which can be automatically provided to caregivers in real-time include: how much assistance does the resident require for activities of daily living, how much assistance, if any, is needed for transfers, what is the resident's primary and secondary mode of transportation, for example, walker, wheel chair, does the resident require intake and output monitoring, is the resident continent or does he/she require briefs, pads, does the resident have a catheter, does the resident have dietary restrictions, does the resident require thick liquids to prevent choking, does the resident wear glasses, does the resident have dentures, does the resident need to have siderails up on bed and if so, what type, does the resident require restraints for safety purposes and if so, what type and frequency, does the resident require turning and repositioning in bed or wheelchair to prevent or assist with healing of pressure ulcers, and if so, what schedule is currently in place, does the resident require Ted hose and if so, what are the scheduling specifications, is the resident at risk for falls, does the resident require splints and if so, what is the current scheduling, does the resident require alarms and if so, what type and schedule, when is the resident to be showered and by whom, what type of restorative care program or plans is/are currently in place to be performed by a Level II Restorative Aide, what type of restorative care program or plans is or are currently in place to be performed by the Level III CNA, what type of restorative care program or plans is or are currently in place to be performed by the Level I licensed therapist, what special devices are currently being used, and what special duties or instructions are to be relayed to CNAs and other direct caregivers daily. Some or all of this and additional information can be provided to caregivers on a real time basis through systems such as those described above in connection with Figs. 1A-1D, or other systems, and may include display of real time information on a display of a computer workstation or on a handheld or portable computer display, automatic generation of a physical assignment sheet, or in other manners as discussed above.

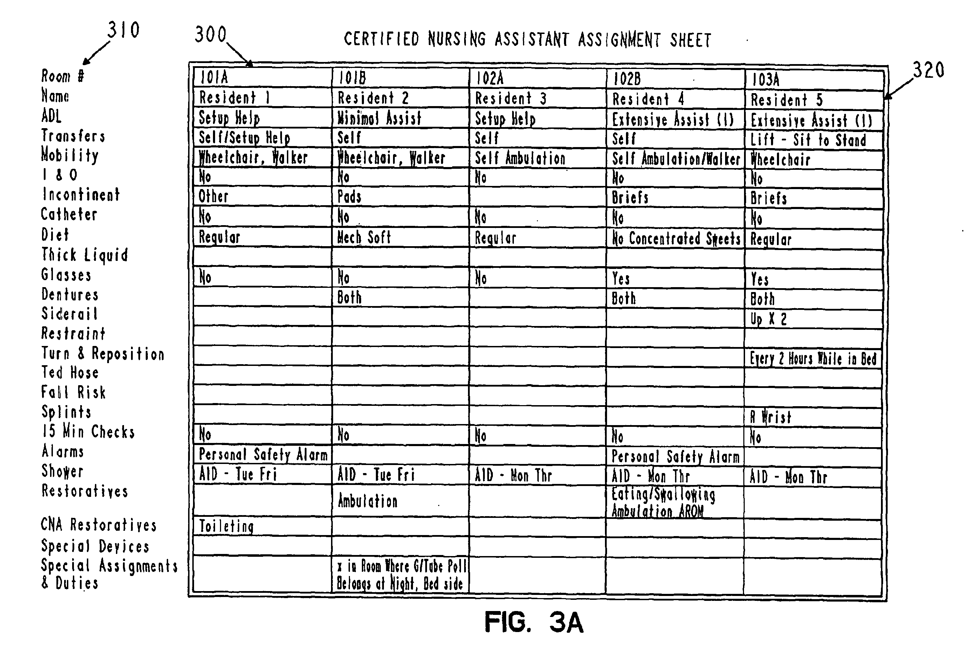

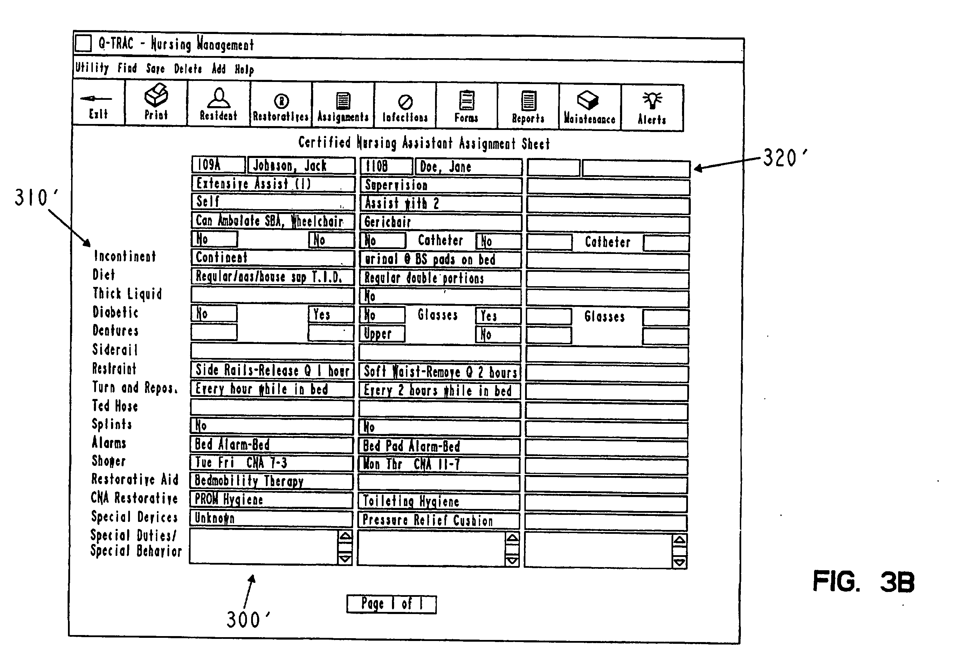

With reference to Figs. 3A and 3B, there is shown exemplary assignment sheets for a grouping of rooms, i.e., 101A, 101B, 102A, 102B, 103A of a long-term care facility. A vertical column 300, 300' of information and/or instructions is provided for each resident. The various types of restorative care program data 310, 310' are listed vertically alongside the column 300, 300' so that the same data is located in the same row 320, 320' for each resident. The information shown on an assignment sheet can include staffing assignments for particular date ranges, particular shift ranges, particular rooms, particular residents, particular caregivers or combinations of these and other categories. The assignment sheet may be created and displayed online, and may be printed and distributed, for example, to appropriate care givers. Also, the assignment sheet may be electronically transmitted to a remote computing device, for example, a handheld device used by a caregiver.

Figs. 3A and 3B shows exemplary assignment sheets in a table format. Additionally or alternatively, sheets can be generated in a variety of other formats for presenting the restorative care program data in easy-to-read fashion. The assignment sheet can be printed automatically and can additionally or alternatively be displayed on a display of a computer workstation or on a handheld or portable computer display or other device, or in other manners as discussed above.

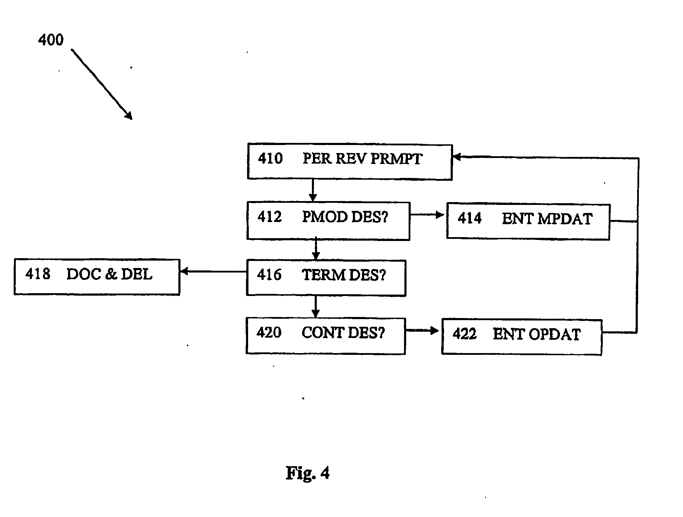

With reference to Fig. 4 there is shown a flow diagram 400 illustrating operation of a restorative care monitoring and update program module according to one embodiment of the present invention. At operation 410 the restorative care monitoring and update program module periodically prompts a user to review the restorative program for a resident, for example, on a monthly basis. It is contemplated that the caregiver could be the user or that the user could be another person. From operation 410 the restorative care monitoring and update program module proceeds to conditional 412.

At conditional 412 the restorative care monitoring and update program module receives and tests input as to whether a caregiver has elected to modify the restorative care program for a resident. If input indicating that the plan is to be modified is received the restorative care monitoring and update program module proceeds to operation 414. At operation 414 the user enters changes or updates to a resident's restorative care program. From operation 414 restorative care monitoring and update program module returns to operation 410 which periodically prompts a user to review the restorative program for a resident as previously described. If input indicating that the program is not to be modified is received the restorative care monitoring and update program module proceeds to conditional 416.

At conditional 416 the restorative care monitoring and update program module receives and tests input as to whether a caregiver has elected to discontinue a restorative care program for a resident. If input indicating that the program is to be discontinued is received the restorative care monitoring and update program module proceeds to operation 418. At operation 418 the reason or reasons why the restorative program was discontinued are documented and the restorative care program is taken out of the system, designated inactive, or deleted. If input indicating that the program is not to be discontinued is received the restorative care monitoring and update program module proceeds to conditional 420.

At conditional 420 the system receives input indicating that a resident's restorative program is to be continued. This input could be the same or similar as input indicating that the discontinuation of the program is not desired, that is, a single input would designate either discontinuation or continuation, or the inputs could be separately received. If input indicating that the resident's restorative program is to be continued is received the restorative care monitoring and update program module proceeds to operation 422.

At operation 422 the restorative care monitoring and update program module can receive optional updates or changes to a resident's restorative care program. From operation 422 the restorative care monitoring and update program module returns to operation 410 where it prompts a user to review the restorative program for a resident as was described above. If no input is received the system can re-cycle through conditionals 412, 416, and 420, can display an overdue prompt or alert or can simply wait for user input.

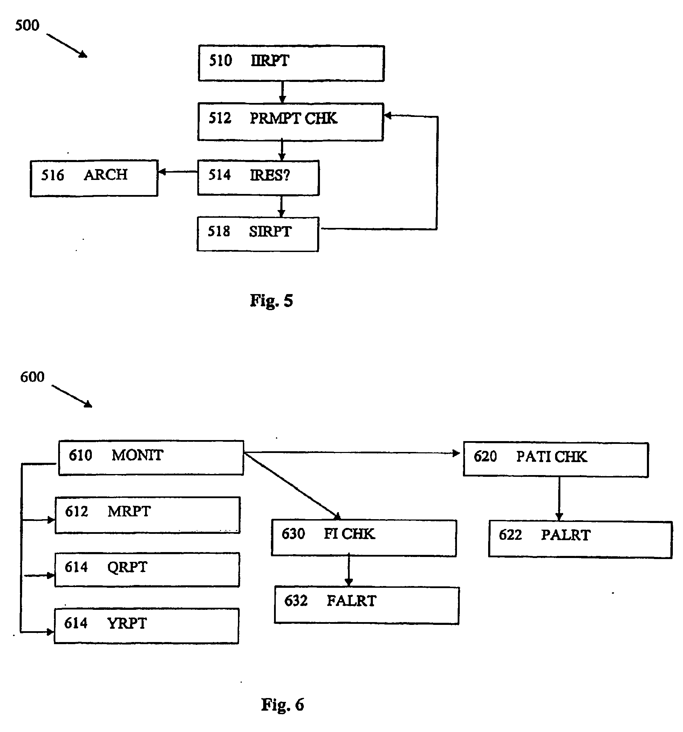

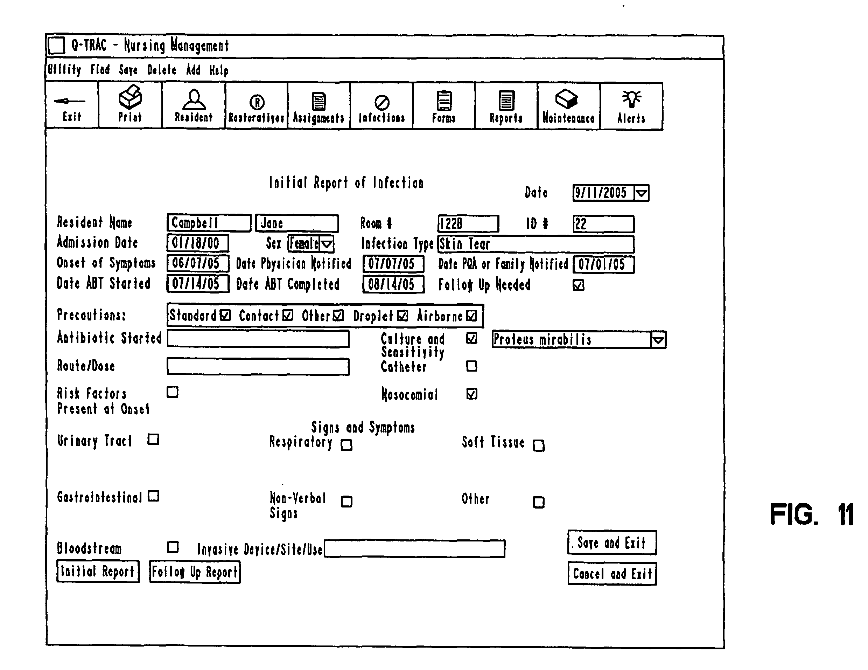

With reference to Fig. 5 there is shown a flow diagram 500 illustrating operation of an infection monitoring program module according to one embodiment of the present invention. Once a resident's infection has been identified by a caregiver, a user can create an initial infection report at operation 510. An exemplary user interface for reporting infections is shown in Fig. 11. The initial infection report is entered by the user and can include, for example, the resident, resident's room number, the type of infection, the date of symptom onset, antibiotic treatment start and end dates, culture and sensitivity, signs and symptoms, lab test results, and additional information. Once the initial infection report is created a record of the input information is saved and a chart copy of the report may be printed, signed by a caregiver such as a licensed nurse, and placed into physical paper file. From operation 510 the infection monitoring program module proceeds to operation 512.

At operation 512 the infection monitoring program module issues a prompt to a caregiver to recheck the infection. This prompt may be based upon a variety of criteria, such as the passage of a certain amount of time indicative of completion of course of antibiotic treatment, for example, the antibiotic end date entered by the user, or based upon standard of care criteria for associated with a type of infection. Once a caregiver has rechecked an infection a follow up report can be prepared. From operation 512, the infection monitoring program module proceeds to conditional 514.

At conditional 514 an input indicating whether the infection is resolved is received and tested. If the infection has resolved the user can enter an indication of this and the infection monitoring program module proceeds to operation 516 where the record of the infection is archived by being removed from the current infections records and added to the infection history records. The infection record remains available for statistical purposes. If the infection has not resolved the user can enter an indication of this and infection monitoring program module proceeds to operation 518.

At operation 518 the user is prompted to enter a supplemental infection report. The supplemental infection report can include the same or similar types of information as the initial infection report or may include additional or less information as may be appropriate. Once the supplemental infection report is entered a record of the input information is saved and a chart copy of the supplemental report may be printed, signed by a caregiver such as a licensed nurse, and placed into physical paper file. After a supplemental infection report has been entered the infection monitoring program module returns to operation 512 where the infection monitoring program module issues a prompt to a caregiver to recheck the infection at an appropriate time as was previously described. In this manner the infection monitoring program module can continue to store and update infection information and provide appropriated caregiver prompts until an infection is resolved.

With reference to Fig. 6 there is shown a flow diagram 600 further illustrating operation of an infection monitoring program module according to one embodiment of the present invention. During operation of the infection monitoring program module the system engages in automated monitoring and reporting indicated at operation 610. The automated monitoring and reporting of operation 610 includes several aspects. As indicated in operation 612, one automated monitoring and reporting aspect is that every month, a database including records of resident infections is queried and a report is created indicating which residents had infections, the type and frequency, as well as the percentages of facility with certain infectious processes, in a monthly, quarterly, and/or yearly format. As indicated in operation 614, another automated monitoring and reporting aspect is that every quarter, a database including records of resident infections is queried and a report is created indicating which residents had infections, the type, and frequency in a monthly format. As indicated in operation 614, a further monitoring and reporting aspect is that every year, a database including records of resident infections is queried and a report is created indicating which residents had infections, the type, and frequency in an annualized format. The time periods for each automated report generation feature can differ and can be deactivated or activated as desired and can also be adjusted as desired.

A further automated monitoring and reporting aspect is indicated at operation 620 where the number and type of infections that a resident has suffered over a given time period are automatically monitored. For example, the system can query a database to identify whether a resident has had three or more of the same types of infectious process within a six month period. The time period and infection count values can differ and can be adjusted to different values. As indicated at operation 622, an alert can be sent to the user when the query indicates that the given criteria are satisfied, for example, that a resident has had three or more of the same types of infectious process within a six month period.

An additional automated monitoring and reporting aspect is indicated at operation 630 where the number and type of infections for all residents at a facility can be automatically monitored. For example, the system can query a database to identify whether the facility has 10% or more of residents with the same type of infection at a particular time or over a particular time range. The time range and resident percentage values can differ and can be altered to various values as desired. Furthermore, the monitoring can be performed for subgroups of all residents at a facility such as all residents of a certain wing, floor or section of the facility.

At operation 632, an alert is sent to the user when the query indicates that the given criteria are satisfied, for example, when the facility has 10% or more of residents with the same type of infection at a particular time. The automated monitoring features of operations 620 and 630 can be performed on a daily, weekly, monthly or quarterly basis or based on another time period or can be initiated based upon a user input.

Fig. 7 is a simplified schematic view of an embodiment of the present invention including an interface for receiving data from or sending data to an external or remote computing device, computer, or computer system 20 over a network 16'. In other embodiments, a network 16' is not utilized and data may be transferred between the two computing devices, computers, or systems 10''' and 20 by other electronic, manual, or conventional methods. In the illustrated embodiment, the external or remote device, computer or system 20 is a minimum data set or MDS system used by a long-term care facility. Certain data fields in the assignment sheet described herein may be linked to the MDS system 20. For example, in the illustrated embodiment, sections C2, D3, G, H, I, L and P of the MDS are linked to the assignment sheet of the device, computer or system 10''' in accordance with the present invention. The resident care information stored and maintained in the device, computer, or system 10''' is continually updated upon changes in residents' condition or level of care. Such continuous updates are then available for monitoring within the device, computer or system 10''' and also for transfer to the external or remote device, computer, or system 20. Such continuous monitoring enables long-term care facilities to react quickly to changes in residents' conditions.

Early detection of and response to such changes helps attain or maintain each resident's well-being at its highest practicable level as may be required by government regulations. Increasing the number of residents functioning at their highest practicable level may facilitate receiving reimbursement or payment for restorative care by long-term care facilities. Restorative care program data from the device, computer or system 10''' may be entered into a remote or external MDS system 20 or similar device, computer or system to support payment or reimbursement of a long term care facility for restorative care provided.

The various embodiments of program modules described above with respect to a single resident, can also operate with respect to all residents at facility or in a given system or database. Alternatively, a module could operate with respect to a subset of all residents at a facility or a set of residents at more than one facility. Furthermore, while some operations and conditionals have illustrated actions and inputs as occurring in series, these acts or inputs could also occur in parallel. For example, when a series of inputs from a user are received, the user could be presented with and select from all choices simultaneously or on a single menu. Additionally, when it is described that a database is queried, alternatives such as setting one or more flags, or keeping count of criteria in separate data structures could be employed.

While the present invention has been illustrated and described in detail in the drawings and foregoing description, the same is to be considered as illustrative and not restrictive in character, it being understood that only an exemplary embodiment has been shown and described and that all changes and modifications that come within the spirit of the present invention are desired to be protected.

## Claims

1. A method for managing restorative care in a long-term care facility with the use of a computer system (10, 10', 10"), the method comprising:
receiving a resident identifier, the resident identifier including an identification number and identifying a resident of a long-term care facility;
creating with a menu driven interface a restorative care program including a description of the resident's mental condition, physical condition or psychosocial condition, a goal for maintaining or improving the resident's condition, a plan for caregivers to achieve the goal, and an indication of the amount of caregiver assistance required by the resident;
assigning one of a plurality of levels to the restorative care program, each of the plurality of levels identifying a different level of care provided by a caregiver of a specific type;
linking the restorative care program to the resident identifier;
storing the resident identifier, level, and restorative care program in a computer-readable medium, each of the plurality of levels being input through menu of a menu driven interface;
repeating the above receiving, creating, assigning, linking, and storing operations for a plurality of residents of a long-term care facility;
receiving data relating to a work shift, a caregiver, a section of a facility, and a grouping of residents including a portion of the plurality of residents;
generating an assignment sheet for a caregiver, the assignment sheet including resident care data for the grouping of residents, and at least a portion of the restorative care programs for each of the plurality of residents of the group of residents including an amount of caregiver assistance required by the residents;
determining through the use of programming logic whether a change in a resident's mental condition, physical condition or psychosocial condition requires a change in the amount of caregiver assistance, including a change to a different one of the plurality of levels identifying the different level of care provided by the caregiver of the specific type, needed by one of the plurality of residents;
**characterized in that** the method is for managing restorative care mandated by government regulations to facilitate reimbursement for the restorative care; **in that** the method includes receiving infection information of the resident and storing an infection threshold in a computer readable medium, the determining step including determining if the infection information exceeds the infection threshold; **in that** the assignment sheet is automatically updated using programming logic if the amount of caregiver assistance needed changes; and **in that** the method further comprises automatically alerting a caregiver to the updated assignment sheet with an alert and automatically alerting a caregiver if the infection information exceeds the infection threshold, the alert being generated by computer programming logic.

2. The method of claim 1 further comprising:
receiving input indicating changes to the restorative care program;
storing the changes in a computer readable medium; and
sending a communication to a caregiver based upon the changes to the restorative care program.

3. The method of either claim 1 or claim 2 further comprising automatically generating a periodic report including the resident identifier, the goal and the accomplishment data.

4. The method of claim 3 wherein the periodic report is generated at least monthly.

5. The method of either claim 3 or claim 4 further comprising sending at least a portion of the accomplishment data in the report to a remote computer.

6. The method of any preceding claim further comprising:
receiving a plurality of infection information inputs;
storing the plurality of infection information inputs; and
automatically sending a caregiver alert when the number of stored infection information inputs exceeds a limit.

7. The method of any preceding claim wherein the assignment sheet is automatically updated if the work shift, caregiver, section of the facility, or grouping of residents changes.

8. The method of any preceding claim wherein the computer system comprises a computer workstation including an input device, the input device comprising one of a computer mouse, stylus, keypad, keyboard, and microphone.

9. The method of any preceding claim wherein the computer system comprises a display, the display being adapted to display the menu.
